# EUROPÄISCHE PATENTSCHRIFT

(11) **EP 1 875 938 B1**
(45) Veröffentlichungstag und Bekanntmachung des Hinweises auf die Patenterteilung: **12.09.2012**
(21) Anmeldenummer: 07013263.4
(22) Anmeldetag: 06.07.2007
(51) Int. Cl.: A61M 16/06

(54) **Gesichtsmaske, insbesondere Inhalationsmaske für die Verwendung mit einem Therapievernebler**
Face mask, in particular inhalation mask for use with a therapy atomiser
Masque facial, en particulier masque d'inhalation pour l'utilisation avec un vaporisateur thérapeutique

(30) Priorität: 06.07.2006 DE 102006031312
(43) Veröffentlichungstag der Anmeldung: 09.01.2008
(73) Patentinhaber: PARI GmbH Spezialisten für effektive Inhalation, 82319 Starnberg (DE)
(72) Erfinder: Kreutzmann, Vera, 82064 Straßlach Dingharting (DE); Rosenbeiger, Sven, 82319 Starnberg (DE); Zimmermann, Johann, Dr., 50126 Bergheim (DE)
(74) Vertreter: HOFFMANN EITLE

(56) Entgegenhaltungen:
- EP-A- 1 582 231
- GB-A- 837 250
- GB-A- 848 215
- US-B1- 6 192 886

## Beschreibung

Die vorliegende Erfindung betrifft eine Gesichtsmaske und insbesondere eine Gesichtsmaske für die Verwendung mit einem Therapievernebler.

Vernebler für therapeutische Zwecke, im Folgenden als "Therapievernebler" bezeichnet, ermöglichen es einem Benutzer ein medikamenthaltiges Aerosol einzuatmen, das zuvor von einem Aerosolgenerator in dem Therapievernebler erzeugt wurde. Zum Einatmen des Aerosols nimmt der Benutzer üblicherweise ein Mundstück, das an dem Therapievernebler angebracht ist, in den Mund und atmet durch das Mundstück das Aerosol ein. Es gibt jedoch Benutzergruppen, bei denen die Verwendung eines Mundstücks zum Einatmen des Aerosols ungeeignet oder sogar unmöglich ist. Für diese Benutzergruppen werden Inhalations- oder Atemmasken eingesetzt, die auf das Gesicht des Benutzers aufgesetzt werden, so dass der Benutzer das über einen Anschlussstutzen zugeführte Aerosol des Therapieverneblers einatmen kann.

Eine derartige Maske ist beispielsweise in dem deutschen Patent DE 101 26 808 C1 beschrieben. Die dort offenbarte Inhalationsmaske umfasst einen Maskengrundkörper, der sich aus einem ersten umlaufenden Bereich zum Anlegen der Maske an das Gesicht eines Benutzers und einem zweiten Bereich zum Anschluss an einen Therapievernebler zusammensetzt. Eine Inhalationsmaske, wie sie in dem deutschen Patent DE 101 26 808 C1 beschrieben ist, wird herkömmlicherweise aus einem Teil aus PP oder PVC hergestellt. Derartige Masken sind insbesondere bezüglich des Tragekomforts und der Dichtfunktion zwischen Maske und Gesicht zu verbessern.

Insbesondere ist bei der Verabreichung von medikamenthaltigem Aerosol darauf zu achten, dass das Aerosol nicht in die Augen des Benutzers gelangen kann, weil dadurch starke Reizungen der Augen ausgelöst werden können. Dementsprechend ist bei den bekannten Masken insbesondere die Abdichtung zwischen Maske und einem oberen Nasenbereich zu verbessern. Unter dem oberen Nasenbereich ist in diesem Zusammenhang der Bereich zu verstehen, der den oberen Teil des Nasenrückens sowie die sich davon erstreckenden Nasenflügel umgibt.

Neben den oben erwähnten Inhalationsmasken sind darüber hinaus aus dem Stand der Technik auch so genannte Schlafapnoemasken bekannt. Eine derartige Schlafapnoemaske ist beispielsweise in dem US Patent US 6,112,746 beschrieben. Die dort offenbarte Gesichtsmaske umfasst ein Maskenkissen zum Anlegen an das Gesicht eines Benutzers. Dieses Kissen weist ferner auch einen Abschnitt auf, der zum Anlegen in einem oberen Nasenbereich des Gesichts ausgelegt ist. Dieses Gesichtskissen umfasst zwei vollständig umlaufende, nach Innen weisende Dichtlippen auf, die derart hintereinander geschaltet sind, dass sie einander überlappen. Unter dem Begriff "nach Innen weisend" ist diesbezüglich folgendes zu verstehen. Der Bereich zum Anlegen an das Gesicht des Benutzers ist ein umlaufender Bereich, der eine geschlossene Querschnittsform mit einer Öffnung in ihrem Inneren beschreibt. "Nach Innen weisend" bedeutet diesbezüglich, dass die Dichtlippen zur Öffnung hinweisen.

Eine ähnliche Gesichtsmaske ist auch aus dem europäischen Patent EP 0 264 772 B1 bekannt. Auch diese Gesichtsmaske weist in einem Abschnitt zum Anlegen an einem oberen Nasenbereich des Gesichts, zwei hintereinander geschaltete, nach Innen weisende Dichtlippen auf, die einander überlappen.

Trotz dieser zwei hintereinander geschalteter Dichtlippen ist die Abdichtung im oberen Nasenbereich insbesondere für die Verwendung mit einem medikamenthaltigen Aerosol, d.h. mit einem Therapievernebler, verbesserungsfähig. Gleichermaßen lässt sich darüber hinaus auch der Tragekomfort noch verbessern. Aus der GB-A-848,215 ist ferner eine Gesichtsmaske mit zwei hintereinander geschalteten Dichtlippen bekannt, bei denen eine nach außen und eine nach innen weist. Beide Dichtlippen erstrecken sich entlang des gesamten Umfangs.

Eine Gesichtsmaske mit den Merkmalen im Oberbegriff von Patentanspruch 1 ist aus der EP-A-1 582 231 bekannt.

Dementsprechend ist es die Aufgabe der vorliegenden Erfindung, die Eingangs erwähnten Gesichtsmasken derart weiterzubilden, dass die Abdichtung zwischen Gesichtsmaske und Gesicht im oberen Nasenbereich sowie der Tragekomfort verbessert sind.

Gelöst wird dieses Problem durch eine Gesichtsmaske mit den Merkmalen des Patentanspruchs 1. Vorteilhafte Weiterbildungen der vorliegenden Erfindung ergeben sich aus den abhängigen Patentansprüchen.

Die vorliegende Erfindung schlägt eine Gesichtsmaske, insbesondere eine Inhalationsmaske für die Verwendung mit einem Therapievernebler, vor, die einen Maskengrundkörper umfasst. Der Maskengrundköper umfasst einen ersten umlaufenden Bereich, der zum Anlegen der Maske an das Gesicht eines Benutzers ausgelegt ist und einen zweiten Bereich zum Anschluss an insbesondere einen Therapievernebler bzw. einen Anschlussstutzen eines solchen. Ein Abschnitt des ersten Bereichs, der zum Anlegen an einen oberen Nasenbereich des Gesichts ausgelegt ist, weist dabei nur zwei hintereinander geschaltete Dichtlippen auf. Diese zwei hintereinander geschalteten Dichtlippen sind erfindungsgemäß durch eine erste nach Innen weisende bzw. innen liegende Dichtlippe und eine zweite nach Außen weisende bzw. außen liegende Dichtlippe gebildet. Dabei ist unter der Begrifflichkeit "Innen" bzw. "Außen", unter Bezug auf die obigen Ausführungen, zu verstehen, dass die nach Innen weisende Dichtlippe nach Innen zur durch den umlaufenden ersten Bereich beschriebenen Öffnung hinweist, wohingegen die zweite nach Außen weisende Dichtlippe von dieser Öffnung weg weist. Durch die erfindungsgemäße Ausgestaltung der Dichtlippen kann erreicht werden, dass sich der erste Bereich gut an unterschiedliche Gesichtskonturen und insbesondere an den oberen Nasenbereich anpasst. Hierfür beschreibt die nach innen weisende Kante der ersten Dichtlippe die Form des oberen Nasenbereichs. Insbesondere der obere Nasenbereich ist von Mensch zu Mensch sehr verschieden, so dass dies auch der schwierigste Bereich der Abdichtung ist. Insbesondere wird durch die vorliegende Erfindung, und insbesondere die Ausgestaltung der Dichtlippen, eine besonders gute Dichtfunktion in diesem Bereich erzielt und es kann daher verhindert werden, dass zum Beispiel ein medikamenthaltiges Aerosol entweicht und in die Augen gelangt, was zu Reizungen der Augen führen kann. Darüber hinaus ist die erfindungsgemäße Gesichtsmaske auch hinsichtlich des Tragekomforts verbessert.

Gemäß der vorliegenden Erfindung erstreckt sich die zweite Dichtlippe im Wesentlichen entlang des gesamten umlaufenden ersten Bereichs. D.h. die zweite Dichtlippe liegt über im Wesentlichen dem gesamten Umfang der Gesichtsmaske, der durch den ersten umlaufenden Bereich gebildet wird am Gesicht des Benutzers an und dichtet die Maske gegenüber dem Gesicht ab.

Unter dem Begriff "im Wesentlichen" ist diesbezüglich zu verstehen, dass sich die Dichtlippe über den größeren Teil des gesamten umlaufenden ersten Bereichs erstreckt, jedoch in einigen Teilbereichen ausgespart sein kann, um zum Beispiel Gurte zum Anlegen der Maske zu befestigen. Vorteilhafterweise ist die erste Dichtlippe hingegen im Wesentlichen nur in dem Abschnitt zum Anlegen in einem oberen Nasenbereich vorgesehen und dient der Verstärkung der Dichtwirkung in diesem Bereich. Neben der erhöhten Abdichtungswirkung trägt dies ferner auch zu einer weiteren Verbesserung des Tragekomforts bei. Ferner umfasst die Gesichtsmaske ein Zungenventil, das ausgeatmete Luft aus der Maske entweichen lässt sowie ein Einströmen von Außenluft in die Maske beim Einatmen verhindert.

Durch die erfindungsgemäße Ausgestaltung einer nach Innen und einer nach Außen weisenden Dichtlippe, überlappen einander die erste und die zweite Dichtlippe im Gegensatz zum oben erwähnten Stand der Technik nicht.

Gemäß einer vorteilhaften Ausführungsform der vorliegenden Erfindung ist die zweite Dichtlippe eine nach Außen gekrümmte Dichtlippe, d.h. sie weist eine nach Außen gekrümmte Form auf. Dies verbessert das enge Anliegen der Gesichtsmaske an dem Gesicht eines Benutzers und damit die Dichtwirkung.

Die erste Dichtlippe ist hingegen als nach Innen vorragender Steg ausgebildet und vorteilhafterweise membranförmig, d.h. dünnwandig (dünner als die zweite Dichtlippe) ausgestaltet, damit sie sich beim Anlegen der Maske nicht unangenehm anfühlt ohne jedoch ihre Dichtwirkung zu verlieren.

Um den Tragekomfort und die Dichtwirkung sowie die Verbindungsmöglichkeit zu einem Anschlussstutzen zu verbessern, besteht die Gesichtsmaske der vorliegenden Erfindung aus zwei unterschiedlichen Materialien, wobei ein erstes Material für den ersten Bereich und ein zweites Material für den zweiten Bereich verwendet wird. Dadurch wird es ermöglicht, den ersten Bereich bezüglich des Tragekomforts und der Dichtwirkung und den zweiten Bereich zum Anschließen an zum Beispiel einem Therapievernebler zu optimieren.

Zu diesem Zweck ist der erste Bereich vorzugsweise aus einem weicheren bzw. elastischeren Material gebildet als der zweite Bereich. Beispielsweise können TPE (Thermoplastische Elastomere) für den ersten abdichtenden Bereich und PP (Polypropylen) für den zweiten Bereich zum Anschluss an einen Therapievernebler verwendet werden.

Um eine solche Gesichtsmaske kostengünstig herzustellen, wird das dem Fachmann im Allgemeinen bekannte Zweikomponentenspritzgießverfahren verwendet, das es ermöglicht, zwei unterschiedliche Komponenten in einem Werkzeug zu formen, so dass ein integral ausgebildetes Produkt erzielt werden kann.

Weitere Vorteile, Merkmale und Ausführungen der vorliegenden Erfindung werden aus der folgenden Beschreibung eines Ausführungsbeispiels unter Bezugnahme auf die Figuren ersichtlich.

### In den Figuren zeigen:

Figur 1 eine perspektivische Ansicht einer erfindungsgemäßen Gesichtsmaske; und

Figur 2 einen Längsschnitt der in Figur 1 dargestellten Maske, ebenfalls in Perspektive.

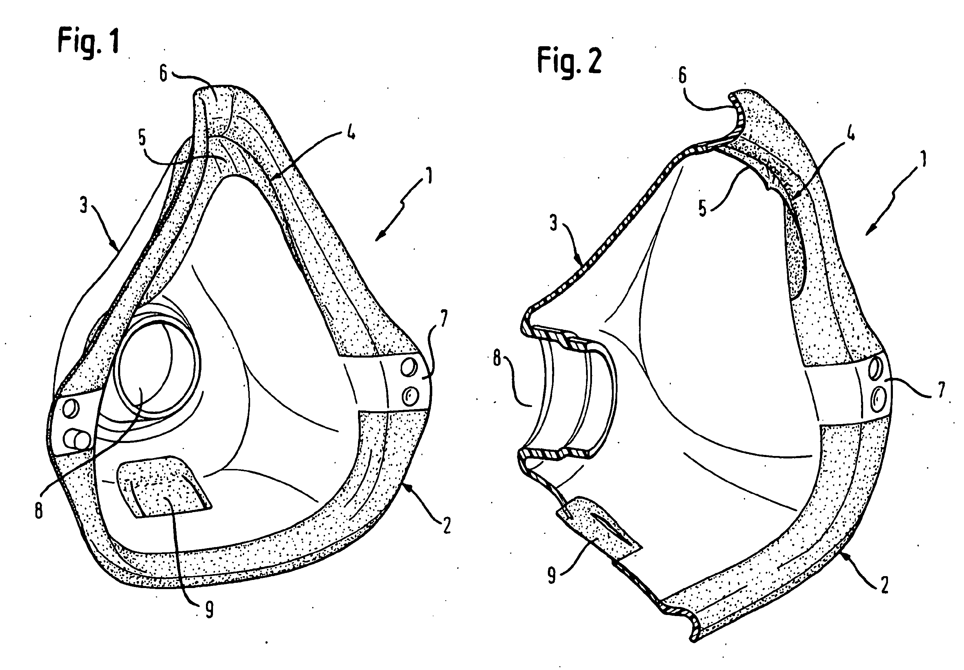

Figur 1 zeigt eine Inhalationsmaske 1 für die Verwendung mit einem Therapievernebler. Die Gesichtsmaske umfasst einen Maskengrundkörper, der sich aus einem ersten umlaufenden Bereich 2 zum Anlegen der Maske an das Gesicht eines Benutzers und einem zweiten Bereich 3 zum Anschluss an einen Therapievernebler zusammensetzt.

Ein Abschnitt 4 des ersten Bereichs 2 ist dabei zum Anlegen in einem oberen Nasenbereich des Gesichts ausgelegt und umfasst zwei hintereinander geschaltete Dichtlippen 5 und 6. Eine der Dichtlippen ist als nach Innen weisende Dichtlippe 5 (erste Dichtlippe) ausgebildet und die andere ist als nach Außen weisende Dichtlippe 6 (zweite Dichtlippe) ausgebildet.

Die nach Außen weisende bzw. außen liegende Dichtlippe 6 erstreckt sich über den gesamten umlaufenden ersten Bereich 2 und liegt im Gebrauchszustand über im Wesentlichen den gesamten Umfang der Dichtlippe 6 am Gesicht eines Benutzers an. Lediglich in einem Bereich 7, in dem die Gesichtsmaske mit Gurten oder Bändern versehen wird um sie am Gesicht des Benutzers festzulegen, ist diese nach Außen weisende Dichtlippe 6 stark verdünnt, wie es insbesondere aus Figur 1 ersichtlich wird. Die außen liegende Dichtlippe 6 weist im Querschnitt eine nach Außen gekrümmte Form auf (siehe Figur 2).

Die nach Innen weisende Dichtlippe 5 ist hingegen nur in dem Abschnitt zum Anlegen im oberen Nasenbereich des Gesichts vorgesehen. Sie ist als dünnwandiger nach Innen vorragender Steg ausgebildet und die nach Innen weisende Kante dieser Dichtlippe 5 beschreibt ungefähr die Form des oberen Nasenbereichs. Insbesondere ist die Dichtlippe 5 lamellenförmig ausgestaltet und dünner als die Dichtlippe 6.

Des Weiteren umfasst die in den Figuren dargestellte Inhalationsmaske einen Anschluss 8 zum Anschließen an einem Anschlussstutzen eines Therapieverneblers. Darüber hinaus ist ein Zungenventil 9 vorgesehen, das die ausgeatmete Luft aus der Maske entweichen lässt. Beim Einatmen hingegen verhindert es ein Einströmen von Außenluft in die Maske.

Wie es in den Zeichnungen dargestellt ist, ist der erste Bereich 2 der aus den zwei Dichtlippen 5 und 6 gebildet ist, aus einem ersten Material gebildet. Auch das Zungenventil 9 ist aus diesem Material gebildet. Der zweite Bereich 3 hingegen ist aus einem anderen Material gebildet. Beispielsweise kann der erste Bereich 2 aus TPE bestehen, wohingegen der zweite Bereich 3 aus PP besteht. Um den Tragekomfort und die Abdichtung zu verbessern, ist es jedoch in jedem Fall bevorzugt, dass das Material des ersten Bereichs 2 weicher und flexibler bzw. elastischer ist als das Material des zweiten Bereichs 3, damit sich die Dichtlippen gut an die Gesichtskontur anpassen werden. Dies führt darüber hinaus dazu, dass der Anschluss 8 zum Anschließen an einen Therapievernebler optimiert und aus einem starren Material gebildet werden kann, so dass der Therapievernebler einerseits leicht angeschlossen und andererseits die Verbindung zum Therapievernebler sicher und zuverlässig aufrechterhalten werden kann.

Obwohl die vorliegende Erfindung anhand einer Inhalationsmaske für die Verwendung mit einem Therapievernebler erläutert wurde, versteht sich, dass die vorliegende Erfindung auch auf andere Gesichtsmasken, wie beispielsweise Schlafapnoe-, Anästhesiemasken oder andere Gesichtsmasken übertragen werden kann. Des Weiteren versteht sich, dass neben den beschriebenen zwei Dichtlippen im oberen Nasenbereich noch weitere Dichtlippen vorhanden sein können. So können beispielsweise neben der nach Außen weisenden Dichtlippe 6 und der nach Innen weisenden Dichtlippe 5 eine weitere innen liegende Dichtlippe oder eine weitere außen liegende Dichtlippe vorgesehen sein. Auch können in anderen Abschnitten des ersten Bereichs 2 weitere zusätzliche Dichtlippen vorgesehen werden.

## Patentansprüche

1. Gesichtsmaske, insbesondere Inhalationsmaske für die Verwendung mit einem Therapievernebler, umfassend
einen Maskengrundkörper, der einen ersten umlaufenden Bereich (2), der zum Anlegen der Maske an das Gesicht eines Benutzers ausgelegt ist, und einen zweiten Bereich (3) zum Anschluss an insbesondere einen Therapievernebler umfasst, wobei ein Abschnitt (4) des ersten Bereichs (2) zum Anlegen in einem oberen Nasenbereich des Gesichts ausgelegt ist; und
zwei hintereinander geschaltete Dichtlippen (5, 6), die durch eine erste nach innen weisende Dichtlippe (5) und eine zweite nach außen weisende Dichtlippe (6) gebildet sind,
wobei sich die zweite Dichtlippe (6) im Wesentlichen entlang des gesamten umlaufenden ersten Bereichs (2) erstreckt,
**dadurch gekennzeichnet, dass** die Gesichtsmaske nur die erste und die zweite Dichtlippe umfasst, wobei
die erste Dichtlippe (5) im Wesentlichen nur in dem Abschnitt (4) zum Anlegen in einem oberen Nasenbereich vorgesehen ist und eine nach innen weisende Kante der ersten Dichtlippe die Form des oberen Nasenbereichs beschreibt und dass die Gesichtsmaske ein Zungenventil (9) umfasst, das ausgeatmete Luft aus der Maske entweichen lässt sowie ein Einströmen von Außenluft in die Maske beim Einatmen verhindert.

2. Gesichtsmaske nach Anspruch 1, bei der sich die erste (5) und die zweite (6) Dichtlippe nicht überlappen.

3. Gesichtmaske nach einem der vorstehenden Ansprüche, bei der die zweite Dichtlippe (6) eine nach außen gekrümmte Form aufweist.

4. Gesichtmaske nach einem der vorstehenden Ansprüche, bei der die erste Dichtlippe (5) als nach innen vorragender Steg ausgebildet ist.

5. Gesichtmaske nach einem der vorstehenden Ansprüche, bei der der erste (2) und der zweite (3) Bereich aus unterschiedlichen Materialien bestehen.

6. Gesichtmaske nach Anspruch 5, bei der der erste Bereich (2) aus einem weicheren Material gebildet ist als der zweite Bereich (3).

7. Gesichtmaske nach Anspruch 5 oder 6, bei der der erste (2) und zweite (3) Bereich im Zweikomponenten-Spritzgießverfahren hergestellt sind.

8. Gesichtsmaske nach Anspruch 5, 6 oder 7, bei der das Zungenventil (9) aus dem gleichen Material wie der erste Bereich gebildet ist.

## Claims

1. Face mask, in particular inhalation mask for use with a therapeutic nebuliser. comprising
a mask base body comprising a first peripheral region (2) which is designed for application of the mask to a user's face, and a second region (3) for connection in particular to a therapeutic nebuliser, wherein a section (4) of the first region (2) is designed for application in an upper nose region of the face; and
two sealing lips (5, 6) one behind the other, which are formed by a first inwardly pointing sealing lip (5) and a second outwardly pointing sealing lip (6),
wherein the second sealing lip (6) extends substantially along the whole of the peripheral first region (2),
**characterised in that** the face mask comprises only the first and second sealing lips, wherein
the first sealing lip (5) is provided substantially only in the section (4) for application in an upper nose region, and an inwardly pointing edge of the first sealing lip describes the shape of the upper nose region, and **in that** the face mask comprises a tongue valve (9) which allows exhaled air to escape from the mask and prevents inflow of external air into the mask during inhalation.

2. Face mask according to claim 1, in which the first (5) and second (6) sealing lips do not overlap.

3. Face mask according to any of the preceding claims, in which the second sealing lip (6) has an outwardly curved shape.

4. Face mask according to any of the preceding claims, in which the first sealing lip (5) is constructed as an inwardly projecting web.

5. Face mask according to any of the preceding claims, in which the first (2) and second (3) regions are made of different materials.

6. Face mask according to claim 5, in which the first region (2) is composed of a softer material than the second region (3).

7. Face mask according to claim 5 or 6, in which the first (2) and second (3) regions are made by two-component injection moulding.

8. Face mask according to claim 5, 6 or 7, in which the tongue valve (9) is made of the same material as the first region.

## Revendications

1. Masque facial, en particulier masque pour inhalation destiné à être utilisé avec un atomiseur thérapeutique, comprenant
un corps de base, comportant une première zone périphérique (2) prévue pour l'application du masque contre le visage d'un utilisateur, et une deuxième zone (3) destinée au raccordement avec un atomiseur thérapeutique en particulier, une partie (4) de la première zone (2) étant prévue pour être appliquée sur une zone nasale supérieure du visage ; et
deux lèvres d'étanchéité (5, 6) disposées l'une derrière l'autre, formées par une première lèvre d'étanchéité (5) dirigée vers l'intérieur et par une deuxième lèvre d'étanchéité (6) dirigée vers l'extérieur,
la deuxième lèvre d'étanchéité (6) s'étendant sensiblement le long de toute la première zone périphérique (2),
**caractérisé en ce que** ledit masque facial ne comprend que la première et la deuxième lèvre d'étanchéité,
la première lèvre d'étanchéité (5) n'étant essentiellement prévue que dans la partie (4) à appliquer sur une zone nasale supérieure, et un bord dirigé vers l'intérieur de la première lèvre d'étanchéité présentant la forme de la zone nasale supérieure, et **en ce que** ledit masque facial comprend une valve à languette (9) qui laisse s'échapper l'air expiré du masque tout en empêchant une pénétration de l'air extérieur dans le masque lors de l'inhalation.

2. Masque facial selon la revendication 1, où la première (5) et la deuxième (6) lèvres d'étanchéités ne se chevauchent pas.

3. Masque facial selon l'une des revendications précédentes, où la deuxième lèvre d'étanchéité (6) présente une forme incurvée vers l'extérieur.

4. Masque facial selon l'une des revendications précédentes, où la première lèvre d'étanchéité (5) est réalisée sous forme de nervure faisant saillie vers l'intérieur.

5. Masque facial selon l'une des revendications précédentes, où la première (2) et la deuxième (3) zones sont composées de matériaux différents.

6. Masque facial selon la revendication 5, où la première zone (2) est formée d'un matériau plus souple que la deuxième zone (3).

7. Masque facial selon la revendication 5 ou la revendication 6, où la première (2) et la deuxième (3) zones sont réalisées au moyen d'un procédé de moulage par injection de deux composants.

8. Masque facial selon la revendication 5, la revendication 6 ou la revendication 7, où la valve à languette (9) est constituée du même matériau que la première zone.
